(19) Europäisches Patentamt
European Patent Office
Office européen des brevets

(11) EP 2 330 347 B1

(12) # EUROPÄISCHE PATENTSCHRIFT

(45) Veröffentlichungstag und Bekanntmachung des Hinweises auf die Patenterteilung:
**26.12.2012 Patentblatt 2012/52**

(51) Int Cl.:
***F23N 1/00** (2006.01)* ***F23N 5/00** (2006.01)*
***G01N 33/22** (2006.01)* ***G01N 33/28** (2006.01)*

(21) Anmeldenummer: **10013484.0**

(22) Anmeldetag: **09.10.2010**

(54) **Verfahren zur Bestimmung des bei der Verbrennung von Brenngas emittierten Kohlenstoffdioxids**

Method for determining the carbon dioxide emitted as a result of the combustion of fuel gas

Procédé de détermination du dioxyde de carbone émis par du gaz combustible lors de la combustion

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.11.2009 DE 102009054316**

(43) Veröffentlichungstag der Anmeldung:
**08.06.2011 Patentblatt 2011/23**

(73) Patentinhaber: **E.ON New Build & Technology GmbH**
**45896 Gelsenkirchen (DE)**

(72) Erfinder: **Schley, Peter, Dr.**
**45133 Essen (DE)**

(74) Vertreter: **Harlacher, Mechthild**
**E.ON Ruhrgas AG**
**Brüsseler Platz 1**
**45131 Essen (DE)**

(56) Entgegenhaltungen:
**EP-A2- 0 939 317 EP-A2- 1 715 339**
**EP-A2- 2 042 850**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 330 347 B1

Printed by Jouve, 75001 PARIS (FR)

## Beschreibung

Die Erfindung betrifft ein Verfahren zur Bestimmung des bei der Verbrennung von Brenngas, insbesondere Erdgas, emittierten Kohlenstoffdioxids ($CO_2$).

Ein Verfahren der eingangs genannten Art ist aus der EP 1715339 A2 bekannt. In der EP 1715339 A2 wird ein Verfahren zur Ermittlung der Anteile biogener und fossiler Kohlenstoffdioxidemissionen beim Betrieb von Verbrennungsanlagen, insbesondere von Anlagen zu Müllverbrennung, beschrieben. Die Anteile biogener und fossiler Kohlenstoffdioxidemissionen werden aus mindestens drei der folgenden Bilanzen bestimmt: der Massebilanz, der Aschebilanz, der Kohlenstoffbilanz, der Energiebilanz, der Bilanz aus Sauerstoffverbrauch und Kohlendioxidproduktion und der Wasserbilanz.

In der EP 2042850 A2 wird ein Verfahren zur Bestimmung des Anteils von Kohlenstoff ($x_c$) in Kohlenwasserstoff-Gemischen beschrieben. Aus dem Anteil an Kohlenstoff in Erdgasen kann nicht das bei der Verbrennung entstehende Kohlenstoffdioxid ($CO_2$) ermittelt werden.

Aus der EP 0939317 A2 geht ein Verfahren zur Bestimmung der Gaszusammensetzung von Brenngas, insbesondere Erdgas hervor. Das Verfahren stellt eine Alternative zur Messung der Gaszusammensetzung mittels kostenaufwendiger Gaschromatographen dar.

Treibhausgase sind gasförmige Stoffe in der Luft, die zum Treibhauseffekt beitragen. Die globale Erwärmung der erdnahen Atmosphäre während der vergangenen Jahrzehnte ist nach dem gegenwärtigen wissenschaftlichen Verständnis auf die Verstär kung des natürlichen Treibhauseffektes durch menschliches Einwirken zurückzuführen. Bei den Treibhausgasen bildet Kohlenstoffdioxid, das u. a. durch die Verbrennung fossiler Brennstoffe in die Atmosphäre gelangt, die mengenmäßig größte Emission.

Mit der EU-Richtlinie 2003/87/EG des europäischen Parlamentes und des Rates vom 13. Oktober 2003 über ein System für den Handel mit Treibhausgasemissionszertifikaten in der Gemeinschaft und der Änderung der Richtlinie 96/61/EG des Rates wurde der EU-Emissionshandel eingeführt, ein marktwirtschaftliches Instrument mit dem Ziel, die Treibhausgasemissionen zu senken und somit die im Kyoto-Protokoll festgelegten Klimaschutzziele zu erreichen. Für die Umsetzung des Emissionshandels wurden die sogenannten "Monitoring Leitlinien" (Entscheidung der Kommission vorn 18/07/2007 zur Festlegung von Leitlinien für die Überwachung und Berichter stattung betreffend Treibhausgasemissionen im Sinne der Richtlinie 2003/87/EG des Europäischen Parlamentes und des Rates) erlassen, in denen die Überwachung und die Berichterstattung der Emissionen von Treibhausgasen geregelt wird.

Der Handel von Zertifikaten für Treibhausgase, insbesondere von $CO_2$-Zertwkaten, ist für Betreiber größerer Verbrennungsanlagen, z. B. Kraftwerksbetreiber, Betreiber von erdgasbetriebenen Verdichterstationen, Industrieuntemehmen etc. inzwischen von großer wirtschaftlicher Relevanz Insofern ist auch die genaue Bestimmung der emittierten $CO_2$-Menge von großer Bedeutung, da sich hieraus direkt die zu leistenden Zertifikatskosten abgeleiten. Kann ein Anlagenbetreiber die emittierte Menge an $CO_2$ nicht mit einer nachgewiesenen Genauigkeit bestimmen, so wird dies mit einem entsprechenden "Unsicherheitszuschlag" berücksichtigt; d. h. die Unsicherheit bei der Mengenbestimmung wird dem Anlagenbetreiber zu Lasten gelegt.

Des Weiteren können Anlagenbetreiber bei einer genauen Kenntnis der emittierten $CO_2$-Masse ihre Prozesse entsprechend optimieren. Beispielsweise kann für einen Betreiber eines Gaskraftwerkes die Kenntnis der $CO_2$-Emissionen einen Einfluss auf die Wahl der eingesetzten Gasqualität und damit auf die Wahl der Bezugsquelle/Lieferanten haben.

Die Ermittlung der Masse der $CO_2$-Emissionen erfolgt gemäß der Entscheidung der EU-Kommission vom 18/VH/2007 zur Festlegung von Leitlinien für die Überwachung und Berichterstattung betreffend Treibhausgasemissionen im Sinne der Richtlinie 2003/87/EG des Europäischen Parlaments und des Rates (Monitoring-Leittinien) nach folgender Berechnungsformel:

$$\mathbf{CO_2\text{-Emissionen}\ [t] = Brennstoffstrom\ [m^3]\ *\ Heizwert\ [GJ/m^3]} \qquad \mathbf{(1)}$$

* Emissionsfaktor [t $CO_2$ / GJ]

Der Heizwert und der Emissionsfaktor sind hierbei stoffspezifische Kenngrößen, die von der chemischen Zusammensetzung des eingesetzten Brennstoffes, z. B. Erdgas abhängen. Die Zusammensetzung der Bestandteile kann in Abhängigkeit von der Bezugsquelle großen Schwankungen unterliegen. Für die Bestimmung des Heiz

wertes und des Emissionsfaktors wird nach dem Stand der Technik eine genaue Analyse des Erdgases benötigt. Die Analyse wird dabei in der Regel Ober einen Gaschromatografen gemessen. Dies kann entweder mittels vor Ort installierter Prozessgaschromatografen erfolgen, oder mittels Laborchromatografen, wobei hier vor Ort in regelmäßigen Abständen eine Probe gezogen wird. Beide Verfahren sind mit erheblichem Aufwand und Kosten verbunden.

Die Messung der $CO_2$-Emissionen bzw. der $CO_2$-Masse im Verbrennungsabgas ist aufgrund der hohen Abgastemperaturen sehr aufwändig und daher unpraktikabel. Ferner sind entsprechende Messgeräte am Markt nicht verfügbar.

Aufgabe der Erfindung ist es demgemäß, hier Abhilfe vorzusehen und ein einfaches und gleichzeitig genaues Verfahren zur Bestimmung des bei der Verbrennung emittierten $CO_2$ zur Verfügung zu stellen.

Gelöst wird diese Aufgabe durch ein Verfahren zur Bestimmung des bei der Verbrennung von Brenngas, insbesondere Erdgas, emittierten $CO_2$, dadurch gekennzeichnet, dass

a) der Brennwert ($H_{sv}$), die Normdichte ($p_n$) und der $CO_2$-Anteil ($x_{co2}$) des Brenngases gemessen oder rechnerisch ermittelt werden,

b) aus den in Verfahrensschritt a) ermittelten Werten in einer Auswerteinheit der CO2-Emissionsfaktor ($E_{CO2}$) rechnerisch bestimmt wird.

Der Brennwert ist in der ISO 6976 definiert.

Als Normdichte ($p_n$) ist die Dichte bei Normbedingungen definiert, d. h.,
Temperatur T = 273,15 K und
Druck p = 101325 Pa = 1013,25 mbar.

Unter Auswerteinheit wird eine beliebige elektronische Recheneinheit, beispielsweise ein PC verstanden.

Bei dem erfindungsgemäßen Verfahren wird das emittierte $CO_2$ auf Basis der Gasbeschaffenheitsgrößen Brennwert ($H_{sv}$), Normdichte ($p_n$) und $CO_2$-Anteil ermittelt, die in der Regel im Rahmen der Abrechnung der gelieferten Energiemenge gemessen werden.

Die hohen Genauigkeitsanforderungen der "Monitoring Leitlinien" werden mit dem Verfahren erfüllt.

Ein großer Vorteil des erfindungsgemäßen Verfahrens besteht darin, dass für die Bestimmung der $CO_2$-Essisionen auf Messanordnungen zurückgegriffen werden kann, die bereits für die Bestimmung der Energiemenge des Brenngases für Abrechnungszwecke vorhanden sind. Dadurch kann die Installation einer zusätzlichen Messtechnik zur Bestimmung der Gaszusammensetzung vermieden werden.

Erfindungsgemäß wird im Verfahrensschritt b) zusätzlich der Heizwert ($H_{iv}$) rechnerisch bestimmt.

Vorzugsweise wird das Brenngas durch einen Volumenzähler geleitet und in Verfahrensschritt a) das Normvolumen (Vn) des zugeführten Brenngases gemessen und im Verfahrensschritt b) die Masse des emittierten $CO_2$ ($m_{CO2}$) rechnerisch bestimmt.

Die Gasbeschaffenheitsgrößen Brennwert, Normdichte und $CO_2$-Anteil können mit verschiedenen Verfahren gemessen werden. Zum einen existieren hierfür klassische Messverfahren wie z. B. Kalorimeter oder Normdichtegeber, zum anderen sind aus der Praxis korrelative Verfahren zur Bestimmung der relevanten Gasbeschaffenheitsgrößen bekannt. Des Weiteren können diese Größen in Gastransportnetzen mittels sogenannter "Brennwertrekonstruktionssystemen" bestimmt werden. Diese sind in der "PTB-Anforderung 7.64. Messgeräte für Gas, Brennwertmessgeräte, Ermittlung von Abrechnungsbrennwerten und weiteren Gasbeschaffenheitsdaten mittels Zustandsrekonstruktion (Dez. 1999)" beschrieben.

Nach der Erfindung wird daher in Verfahrensschritt a) der Brennwert ($H_{sv}$), die Normdichte ($p_n$) und der $CO_2$-Anteil ($x_{co2}$) des Brenngases rechnerisch auf Basis eines Brennwertrekonstruktionssystems ermittelt.

Vorzugsweise wird im Verfahrensschritt b) die Masse des emittierten$CO_2$ ($m_{CO2}$) unter Verwendung der Formeln (1) bis (18) berechnet.

Die Erfindung schlägt weiter vor, die Koeffizienten a, b, c, d in den Formeln (10), (11), (12) und (13) aus Messwerten mit mehreren Referenzgasen bekannter Zusammensetzung zu bestimmen.

Erfindungsgemäß werden aus einer Brenngasleitung (1) kontinuierlich ein Teil-Brenngasstrom abgezweigt und die Messungen des Brennwert ($H_{sv}$), der Normdichte ($p_n$) und des $CO_2$-Anteils ($x_{co2}$) im Verfahrensschritt a) in dem Teil-Brenngasstrom durchgeführt.

Bei Ferngastransportleitungen, in denen Erdgas mit Drücken über 50 bar transportiert wird, besteht im Rahmen der Erfindung die Möglichkeit, den Druck des Teil-Brenngasstromes im Wesentlichen auf Atmosphärendruck zu reduzieren.

Im Folgenden wird das erfindungsgemäße Verfahren anhand der Zeichnung im Detail beschrieben. Die Zeichnung zeigt in:

Fig. 1 die Differenz von Normdichte des Kohlenwasserstoffgases ($p_{n,ch}$) und Normdichte von Methan ($p_{n,ch4}$) in kg/m$^3$ als Funktion der Differenz von Brennwert (volumenbezogen) des Kohlenwasserstoffgases ($H_{sv,ch}$) und Brennwert von Methan ($H_{sv,ch4}$) in MJ/m$^3$;

Fig. 2 die Differenz von Heizwert (molar) des Kohlenwasserstoffgases (Him,ch) und Heizwert (molar) von Methan ($H_{im,ch4}$) in kJ/mol als Funktion der Differenz von Brennwert (volumenbezogen) des Kohlenwasserstoffgases ($H_{sv,ch}$) und Brennwert (volumenbezogen) von Methan ($H_{sv,ch4}$) in MJ/m$^3$;

Fig. 3 die Differenz von Heizwert (volumenbezogen) des Kohlenwasserstoffgases ($H_{iv,ch}$) und Heizwert (volumenbezogen) von Methan ($H_{iv,ch4}$) in $MJ/m^3$ als Funktion der Differenz von Brennwert (volumenbezogen) des Kohlenwasserstoffgases ($H_{sv,ch}$) und Brennwert (volumenbezogen) von Methan ($H_{sv,ch4}$) in $MJ/m^3$;

Fig. 4 die Differenz der mittleren Anzahl der Kohlenstoffatome pro Molekül des Kohlenwasserstoffgases und eins als Funktion der Differenz von Brennwert (volumenbezogen) des Kohlenwasserstoffgases ($H_{sv,ch}$) und Brennwert (volumenbezogen) von Methan ($H_{sv,ch4}$) in in $MJ/m^3$;

Fig. 5 relative Abweichungen von $CO_2$-Emissionsfaktoren, die a) aus Abrechnungsgrößen und b) aus Gasanalysen berechnet wurden für 210 Gasanalysen (Abszisse: $H_{sv}$ in in $MJ/m^3$);

Fig. 6 relative Abweichungen von Heizwerten ($H_{iv}$), die a) aus Abrechnungsgrößen und b) aus Gasanalysen berechnet wurden für 210 Gasanalysen (Abszisse: $H_{sv}$ in in $MJ/m^3$);

Fig. 7 ein Ausführungsbeispiel einer Anordnung zur Durchführung des Verfahrens zur Bestimmung des bei der Verbrennung eines Brennsgases emittierten $CO_2$.

Für das Kohlenwasserstoffgas CH lassen sich folgende einfache Korrelationen aufstellen, die grafisch in den Fig. 1 bis 4 dargestellt sind.

Der für die Anwendung von Gleichung (1) benötigte $CO_2$-Emissionsfaktor ist über das Verhältnis der bei der Verbrennung entstehenden Masse an $CO_2$ und der eingesetzten Energiemenge des Brennstoffes definiert:

$$E_{CO2} = \frac{m_{CO2}}{Q_E} \qquad (2)$$

Die Masse an $CO_2$ ergibt sich aus einer Bilanzierung der Kohlenstoffatome gemäß Gleichung (3) unter der Annahme einer stöchiometrischen Verbrennung:

$$m_{CO2} = n \cdot M_{CO2} \cdot \sum_i x_i \cdot nC_i \qquad (3)$$

Die Energiemenge des Brennstoffes - in diesem Fall Erdgas - ergibt sich aus dem Produkt von volumenbezogenen Heizwert $H_{iv}$ und Normvolumen bzw. in molaren Größen ausgedrückt aus molarem Heizwert $H_{im}$ und Stoffmenge n:

$$Q_E = H_{iv} \cdot V_n = H_{im} \cdot n \qquad (4)$$

Durch Einsetzen der Gleichungen (3) und (4) in Gleichung (2) ergibt sich für den spezifischen $CO_2$-Emissionsfaktor:

$$E_{CO2} = \frac{M_{CO2}}{H_{im}} \cdot \sum_i x_i \cdot nC_i \qquad (5)$$

Auf Basis einer detaillierten Gasanalyse lässt sich Gleichung (4) direkt anwenden. Die Berechnung des Heizwertes erfolgt dabei nach ISO 6976 "Natura) Gas - Calculation of Calorific Values, Density, Relative Density and Wobbe Index from Composition. International Standard ISO 6976, second edition 1995-12-01, corrected and reprinted 1996-02-01 ". Steht nun als Information keine vollständige Analyse des Erdgases zur Verfügung, sondern nur die für die Abrechnung benötigten Gasbeschaffenheitsgrößen ($H_{sv}$, $p_n$, $x_{CO2}$), so lässt sich der $CO_2$-Emissionsfaktor nach dem im folgenden beschriebenen Korrelationsverfahren berechnen.

Erdgas besteht im Wesentlichen aus Stickstoff, Kohlenstoffdioxid sowie Kohlenwasserstoffen - vorwiegend aus den Alkanen Methan bis Hexan. Die Kohlenwasserstoffe werden im Folgenden zusammengefasst und durch den Index CH gekennzeichnet. Aus der Bilanz der Stoffmenge ergibt sich für den Stoffmengenanteil des Kohlenwasserstoff-

gases:

$$x_{CH} = 1 - x_{N2} - x_{CO2} \quad (6)$$

Unter Vernachlässigung des Realgasverhaltens, das bei Normbedingungen nur einen geringfügigen Einfluss hat, lässt sich die Normdichte $p_n$ in Abhängigkeit von $X_{N2}$, $X_{CO2}$ und $X_{CH}$ wie folgt darstellen:

$$\rho_n = x_{N2} \cdot \rho_{n,N2} + x_{CO2} \cdot \rho_{n,CO2} + x_{CH} \cdot \rho_{n,CH} \quad (7)$$

Da der Stickstoff und der Kohlenstoffdioxid keinen Beitrag zum Brennwert liefern, ergibt sich für den volumenbezogen Brennwert:

$$H_{sv} = x_{CH} \cdot H_{sv,CH} \quad (8)$$

In ähnlicher Weise lässt sich die Anzahl der Kohlenstoffatome pro Molekül beschreiben:

$$\sum_i x_i \cdot nC_i = x_{CH} \cdot nC_{CH} + x_{CO2} \cdot nC_{CO2} = x_{CH} \cdot nC_{CH} + x_{CO2} \quad (9)$$

Für das Kohlenwasserstoffgas CH lassen sich folgende einfache Korrelationen aufstellen, die grafisch in den Fig. 1 bis 4 dargestellt sind:

Die Normdichte in Abhängigkeit des volumenbezogenen Brennwertes:

$$\rho_{n,CH} - \rho_{n,CH4} = a(H_{sv,CH} - H_{sv,CH4}) \quad (10)$$

mit a = 0,02102 [kg/MJ]

Der molare Heizwert in Abhängigkeit des volumenbezogenen Brennwertes:

$$H_{im,CH} - H_{im,CH4} = b(H_{sv,CH} - H_{sv,CH4}) \quad (11)$$

mit b = 20,726 [$10^{-3} \cdot m^3$/mol]

Der volumenbezogene Heizwert in Abhängigkeit des volumenbezogenen Brennwertes:

$$H_{iv,CH} - H_{iv,CH4} = c(H_{sv,CH} - H_{sv,CH4}) \quad (12)$$

mit c = 0,93378 [$10^{-3} \cdot m^3$/Mol]

Die mittlere Anzahl der Kohlenstoffatome pro Molekül in Abhängigkeit des volumenbezogenen Brennwertes:

$$nC_{CH} - 1 = d(H_{sv,CH} - H_{sv,CH4}) \quad (13)$$

mit d = 0,03329 [$m^3$/MJ]

Die Gleichungen (6), (7), (8), und (10) bilden ein Gleichungssystem, das sich so auflösen lässt, dass $x_{CH}$ in Abhängigkeit der bekannten Abrechnungsgrößen $H_{sv}$, $\rho_n$, $X_{CO2}$ direkt berechnet werden kann:

$$x_{CH} = \frac{\rho_n - \rho_{n,N2} - (\rho_{n,CO2} - \rho_{n,N2}) \cdot x_{CO2} - a \cdot H_{sv}}{\rho_{n,CH4} - \rho_{n,N2} - a \cdot H_{sv,CH4}} \qquad (14)$$

Entsprechend kann nun $nC_{CH}$ aus Gleichung (13) unter Verwendung von Gleichung (8) bestimmt werden:

$$nC_{CH} = d\left(\frac{H_{sv}}{x_{CH}} - H_{sv,CH4}\right) + 1 \qquad (15)$$

Der molare Heizwert des Kohlenwasserstoffgases $H_{im,CH}$ ergibt sich aus Gleichung (11) unter Verwendung von Gleichung (8) zu

$$H_{im,CH} = b\left(\frac{H_{sv}}{x_{CH}} - H_{sv,CH4}\right) + H_{im,CH4} \qquad (16)$$

Wird Gleichung (5) nun unter Verwendung der Beziehung $H_{im} = x_{CH} \cdot H_{im,CH}$ sowie Gleichung (9) wie folgt umgeformt:

$$E_{CO2} = \frac{M_{CO2}}{x_{CH} \cdot H_{im,CH}} \cdot (x_{CH} \cdot nC_{CH} + x_{CO2}) \qquad (17)$$

so kann der $CO_2$-Emissionsfaktor nach anwenden der Gleichungen (14), (15) und (16) direkt berechnet werden.

Des Weiteren wird für die Bestimmung der $CO_2$-Emission gemäß Gleichung (1) noch der volumenbezogene Heizwert benötigt. Dieser ergibt sich unter Verwendung der Beziehung $H_{iv} = x_{CH} \cdot H_{iv,CH}$ aus Gleichung (12) wie folgt:

$$H_{iv} = x_{CH} \cdot \left\{ c\left(\frac{H_{sv}}{x_{CH}} - H_{sv,CH4}\right) + H_{iv,CH4} \right\} \qquad (18)$$

Zur Entwicklung und Bewertung des Korrelationsverfahrens wurden 210 Gasanalysen von typischen Erdgasen betrachtet. Fig. 5 zeigt einen Vergleich der Emissionsfaktoren, die nach dem oben beschriebenen Verfahren aus Basis von Abrechnungsgrößen bzw. direkt aus der Gasanalyse berechnet wurden. Es ergeben sich max. Abweichungen von ±0,035 %, in der Regel betragen die Abweichungen weniger als ±0,02 %.

Ein entsprechender Vergleich für den volumenbezogenen Heizwert ist in Fig. 6 gezeigt. Hier ergeben sich max. Abweichungen von ± 0,0025 %

Eine Anordnung zur Durchführung des erfindungsgemäßen Verfahrens zur Bestimmung der Menge an emittiertem $CO_2$ ist in Fig. 7 schematisch dargestellt.

Von einer Brenngasleitung 1, die als Ferngasleitung für Erdgas ausgebildet ist, zweigt eine Probennahmeleitung 2 zu Entnahme eines Teil-Brenngasstromes ab. In der Probenentnahmeleitung 2 ist ein Druckreduzierungsgerät 3 installiert. In der Brenngasleitung 1 ist ein Gaszähler 4 zur Bestimmung des Normvolumens (Vn) angeordnet. Ein Messgerät 5 dient zur Bestimmung von Brennwert ($H_{sv}$), Normdichte ($p_n$) und des $CO_2$-Anteils ($X_{CO2}$) im Brenngas. Eine Auswerteeinheit 6 dient zur Bestimmung der Masse an emittiertem $CO_2$ ($m_{CO2}$), d. h. der der Menge an $CO_2$, welche bei der Verbrennung des Brenngases entsteht.

Aus einer Brenngasleitung 1 wird kontinuierlich ein Teilstrom des Erdgases entnommen und mittels des Druckreduzierungsgerätes 3 auf etwa 20-100 mbar, d. h. im wesentlichen auf Atmosphärendruck entspannt und einem Messgerät 5, hier einem korrelativen Messgerät, zugeführt. Aus den Messgrößen Brennwert ($H_{sv}$), Normdichte ($p_n$) und

$CO_2$-Anteil ($x_{CO2}$) werden der $CO_2$-Emissionsfaktor ($E_{CO2}$) und der Heizwert ($H_i$) nach dem zuvor beschriebenen Verfahren berechnet. Zusätzlich wird das Normvolumen (Vn) über den Gaszähler 4, hier einem Turbinenradzähler, gemessen. Aus diesen Kenngrößen wird dann die Masse an $CO_2$, die bei der Verbrennung des Brenngases entsteht und in die Luft emittiert, berechnet.

## Patentansprüche

1. Verfahren zur Bestimmung des bei der Verbrennung von Brenngas, insbesondere Erdgas, emittierten Kohlenstoffdioxids $E_{CO2}$,
**dadurch gekennzeichnet, dass**

a) der Brennwert ($H_{sv}$), die Normdichte ($p_n$) und der $CO_2$-Anteil ($x_{co2}$) des Brenngases gemessen oder rechnerisch ermittelt werden,
b) aus den im Verfahrensschritt a) ermittelten Werten in einer Auswerteinheit (6) der $CO_2$-Emissionsfaktor ($E_{co2}$) mittels der Formeln

$$x_{CH} = \frac{\rho_n - \rho_{n,N2} - (\rho_{n,CO2} - \rho_{n,N2}) \cdot x_{CO2} - a \cdot H_{sv}}{\rho_{n,CH4} - \rho_{n,N2} - a \cdot H_{sv,CH4}} \quad (14)$$

$$nC_{CH} = d\left(\frac{H_{sv}}{x_{CH}} - H_{sv,CH4}\right) + 1 \quad (15)$$

$$H_{im,CH} = b\left(\frac{H_{sv}}{x_{CH}} - H_{sv,CH4}\right) + H_{im,CH4} \quad (16)$$

$$E_{CO2} = \frac{M_{CO2}}{x_{CH} \cdot H_{im,CH}} \cdot ( \;_{CH} \cdot nC_{CH} + x_{CO2}) \quad (17)$$

rechnerisch bestimmt wird,
wobei
$\rho_{n.N2}$ *die Normdiche von Stickstroff,*
$P_{n.cha}$ *die Normdichte von Methan ,*
$\rho_{n.co2}$ *die Normdichte von Kohlenstoff dioxid,*
$\rho_{n \cdot N2}$ *die Normdichte von Stickstoff,*
$\alpha$ = 0,02102 *kg/MJ,*
$H_{sv.CH4}$ *der Brennwert von Methan,*
$_nC_{CH}$ *die mittlere Anzahl derKohlenstoffalome,*
$x_{CH}$ *der Stoffmengenateil des Kohlenwasserstoffgases, d* = 0,03329 *$m^3$/MJ,*
$H_{im}$*.CH der molare Heizwert des Kohlenwasserstoffgases,* $H_{in}CH_1$ *der molare Heizwert von Methan,*
*b* = 20,72610$^{-3}$ *$m^3$ / mol,*
$M_{CO2}$ *die molare $CO_2$,*
*ist.*

2. Verfahren nach Anspruch 1,
**dadurch gekennzeichnet, dass** im Verfahrensschritt b) zusätzlich der Heizwert ($H_{iv}$) mittels der Formel

$$H_{iv} = x_{CH} \cdot \left\{ c\left( \frac{H_{sv}}{x_{CH}} - H_{sv,CH4} \right) + H_{iv,CH4} \right\} \qquad (18)$$

rechnerisch bestimmt wird,
wobei
$c$ = 0,93378 $10^{-3} m^3 / mol$,
*$H_{iv}$,$CH_4$ der volumenbezogene Heizwert von Methan ist.*

3. Verfahren nach Anspruch 1 oder 2,
**dadurch gekennzeichnet, dass** das Brenngas durch einen Volumenzähler geleitet und das Normvolumen (Vn) des zugeführten Brenngases gemessen und im Verfahrensschritt b) die Masse des emittierten $CO_2$ ($m_{co2}$) nach der Formel

$$\textbf{CO}_2\textbf{-Emissionen [t] = Brennstoffstrom [m}^3\textbf{] * Heizwert (H}_{iv}\textbf{) [GJ/m}^3\textbf{]} \qquad (1)$$

* Emissionsfaktor($E_{ca2}$) [t $CO_2$ / GJ]
rechnerisch bestimmt wird.

4. Verfahren nach einem der Ansprüche 1 bis 3,
**dadurch gekennzeichnet, dass** im Verfahrensschritt a) der Brennwert ($H_{sv}$) , die Normdichte ($p_n$) und der $CO_2$-Anteil ($x_{co2}$) des Brenngases rechnerisch auf Basis eines Brennwertrekonstruktionssystems ermittelt wird.

5. Verfahren nach einem der Ansprüche 1 bis 4,
**dadurch gekennzeichnet, dass** aus einer Brenngasleitung (1) kontinuierlich ein Teil-Brenngasstrom abgezweigt wird und dass die Messungen des Brennwerts ($H_{sv}$), der Normdichte ($p_n$) und des $CO_2$-Anteils ($x_{co2}$) im Verfahrensschritt a) in dem Teil-Brenngasstrom durchgeführt werden.

6. Verfahren nach Anspruch 5,
**dadurch gekennzeichnet, dass** der Druck des Teil-Brenngasstromes im Wesentlichen auf Atmosphärendruck reduziert wird.

## Claims

1. Method for determining the carbon dioxide ($E_{co2}$) emitted during the combustion of fuel gas, in particular natural gas, **characterised in that**

a) the higher heating value ($H_{sv}$), the standard density ($\rho_n$) and the $CO_2$ concentration ($x_{co2}$) of the fuel gas are measured or calculated,
b) from the values determined in the first step a), the $CO_2$ emission factor ($E_{co2}$) is calculated in an evaluation unit (6) using the formulae

$$x_{CH} = \frac{\rho_n - \rho_{n,N2} - (\rho_{n,CO2} - \rho_{n,N2}) \cdot x_{CO2} - a \cdot H_{sv}}{\rho_{n,CH4} - \rho_{n,N2} - a \cdot H_{sv,CH4}} \qquad (14)$$

$$nC_{CH} = d\left( \frac{H_{sv}}{x_{CH}} - H_{sv,CH4} \right) + 1 \qquad (15)$$

$$H_{im,CH} = b\left(\frac{H_{sv}}{x_{CH}} - H_{sv,CH4}\right) + H_{im,CH4} \quad (16)$$

$$E_{CO2} = \frac{M_{CO2}}{x_{CH} \cdot H_{im,CH}} \cdot \left(x_{CH} \cdot nC_{CH} + x_{CO2}\right) \quad (17)$$

where
$\rho_{n',N2}$ is the standard density of nitrogen,
$\rho_{n',CH4}$ *is* the standard density of methane,
$\rho_{n'}CO_2$ is the standard density of carbon dioxide,
$\rho_{n,}N_2$ is the standard density of nitrogen,
a = 0.02102 kg/MJ,
$H_{sv,CH4}$ is the higher heating value of methane,
$nC_{CH}$ is the average number of carbon atoms,
$x_{CH}$ is the mole fraction of the hydrocarbon gas,
d = 0.03329 $m^3$/MJ,
$H_{im'}CH$ is the molar lower heating value of the hydrocarbon gas,
$H_{im',CH4}$ is the molar lower heating value of methane,
b = 20.726 $10^{-3}$ $m^3$/mol,
$M_{co2}$ is the molar mass of $CO_2$

**2.** Method according to claim 1,
**characterised in that** in step b) the lower heating value ($H_{iv}$) is also calculated using the formula

$$H_{iv} = x_{CH} \cdot \left\{ c\left(\frac{H_{sv}}{x_{CH}} - H_{sv,CH4}\right) + H_{iv,CH4} \right\} \quad (18)$$

where
c = 0.93378 $10^{-3}$ $m^3$/mol
$H_{iv,CH4}$ is the volume-related lower heating value of methane

**3.** Method according to claim 1 or 2,
**characterised in that** the fuel gas is passed through a flowmeter and the standard volume ($V_n$) of the supplied fuel gas is measured and in step b) the mass of the emitted $CO_2$ ($m_{co2}$) is calculated according to the formula

$CO_2$ emissions [t] = fuel gas stream [m³] * lower heating value ($H_{iv}$) [GJ/m³](1)

* emission factor ($E_{co2}$) [t $CO_2$ / GJ].

**4.** Method according to any one of claims 1 through 3,
**characterised in that** in the step a) the higher calorific value ($H_{sv}$), the standard density (□$_n$) and the $CO_2$ concentration ($X_{co2}$) of the fuel gas is calculated on the basis of a higher heating value reconstruction system.

**5.** Method according to any one of claims 1 through 4,
**characterised in that** a partial fuel gas stream is continuously withdrawn from a fuel gas line (1) and that the measurements of the higher heating value ($H_{sv}$), the standard density (□$_n$) and the $CO_2$ concentration ($X_{co2}$) are carried out in step a) in the partial fuel gas stream.

**6.** Method according to claim 5,
**characterised in that** the pressure of the partial fuel gas stream is largely reduced to atmospheric pressure.

## Revendications

**1.** Procédé pour déterminer le dioxyde de carbone ($E_{co2}$) émis lors de la combustion d'un gaz combustible, en particulier de gaz naturel, **caractérisé par le fait que**

a) le pouvoir calorifique supérieur ($H_{sv}$), la masse volumique normale ($\rho_n$) et la part du $CO_2$ du gaz combustible sont mesurés ou déterminés par voie de calcul,
b) à partir des valeurs calculées dans l'étape a), le facteur d'émission de $CO_2$ ($E_{co2}$) est déterminé par voie de calcul dans une unité de traitement de données suivant les formules :

$$x_{CH} = \frac{\rho_n - \rho_{n,N2} - (\rho_{n,CO2} - \rho_{n,N2}) \cdot x_{CO2} - a \cdot H_{s,v}}{\rho_{n,CH4} - \rho_{n,N2} - a \cdot H_{sv,CH4}} \quad (14)$$

$$nC_{CH} = d\left(\frac{H_{sv}}{x_{CH}} - H_{sv,CH4}\right) + 1 \quad (15)$$

$$H_{im,CH} = b\left(\frac{H_{sv}}{x_{CH}} - H_{sv,CH4}\right) + H_{im,CH4} \quad (16)$$

$$E_{CO2} = \frac{M_{CO2}}{x_{CH} \cdot H_{im,CH}} \cdot (xCH \cdot nC_{CH} + x_{CO2}) \quad (17),$$

où
$\rho_{n,N2}$ est la masse volumique normale de l'azote,
$\rho_{n,ch4}$ est la masse volumique normale du méthane,
$p_{n,CO2}$ est la masse volumique normale du dioxyde de carbone,
a = 0,02102 kg / MJ,
$H_{sv,CH4}$ est le pouvoir calorifique supérieur du méthane,
$nC_{CH}$ est le nombre moyen des atomes de carbone,
$X_{CH}$ est la part de la quantité de matière du gaz hydrocarburé,
d = 0,03329 $m^3$/MJ,
$H_{im,CH}$ est le pouvoir calorifique inférieur molaire du gaz hydrocarburé,
$H_{im,CH4}$ est le pouvoir calorifique inférieur molaire du méthane,
b = 20,726 $10^{-3}$ $m^3$/ mol,
$M_{co2}$ est la masse molaire du $CO_2$.

**2.** Procédé suivant la revendication 1,
**caractérisé par le fait que** dans l'étape b) est déterminé par voie de calcul en plus le pouvoir calorifique inférieur ($H_{iv}$) suivant la formule :

$$H_{iv} = x_{CH} \cdot \left\{c\left(\frac{H_{sv}}{x_{CH}} - H_{sv,CH4}\right) + H_{iv,CH4}\right\} \quad (18),$$

où
c = 0,93378 $10^{-3}$ $m^3$ / mol,
$H_{iv,CH4}$ est le pouvoir calorifique inférieur volumique du méthane.

**3.** Procédé suivant l'une des revendications 1 ou 2,
**caractérisé par le fait que** le gaz combustible passe par un compteur volumétrique, que le volume normale (Vn) du gaz combustible alimenté est mesuré et que dans l'étape b) est déterminé par voie de calcul la masse du $CO_2$ ($m_{co2}$) émis suivant la formule :

$$\text{Emissions de } CO_2 \text{ [t]} = \text{flux du gaz combustible [m}^3\text{]} * \text{PCI } (H_{iv}) \text{ [GJ/m}^3\text{]} \quad (1)$$

* facteur d'émission ($E_{co2}$) [t $CO_2$ / GJ].

**4.** Procédé suivant l'une des revendications 1 à 3,
**caractérisé par le fait que** dans l'étape a) sont déterminés par voie de calcul, sur la base d'un système de reconstruction du pouvoir calorifique supérieur, le pouvoir calorifique supérieur ($H_{sv}$), la masse volumique normale ($\rho_n$) et la part du $CO_2$ ($X_{CO2}$) du gaz combustible.

**5.** Procédé suivant l'une des revendications 1 à 4,
**caractérisé par le fait qu'**un flux partiel du gaz combustible est dévié d'une conduite de gaz combustible (1) et que les mesures du pouvoir calorifique supérieur ($H_{sv}$), de la masse volumique normale ($\rho_n$) et de la part du $CO_2$ ($x_{CO2}$) dans l'étape a) sont réalisées dans ledit flux partiel du gaz combustible.

**6.** Procédé suivant la revendication 5,
**caractérisé par le fait que** la pression du flux partiel du gaz combustible est essentiellement réduite à la pression atmosphérique.

Fig. 1

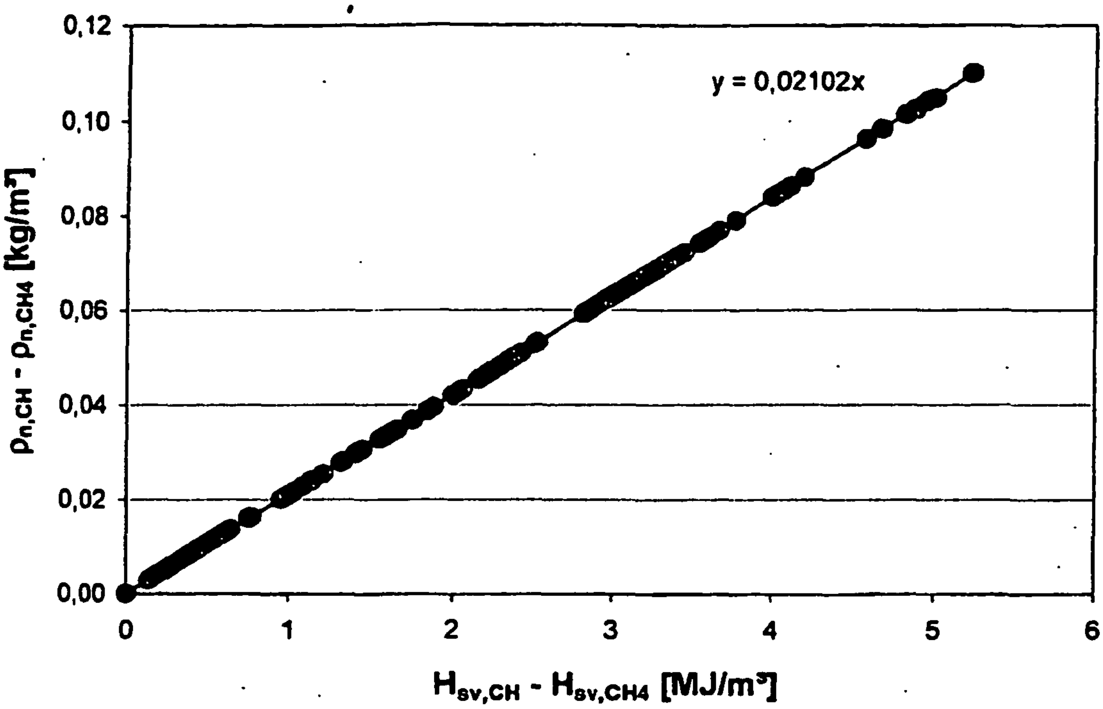
y = 0,02102x
0,12
0,10
0,08
0,06
0,04
0,02
0,00
0
1
2
3
4
5
6
ρn,CH - ρn,CH4 [kg/m³]
Hsv,CH - Hsv,CH4 [MJ/m³]

Fig. 2

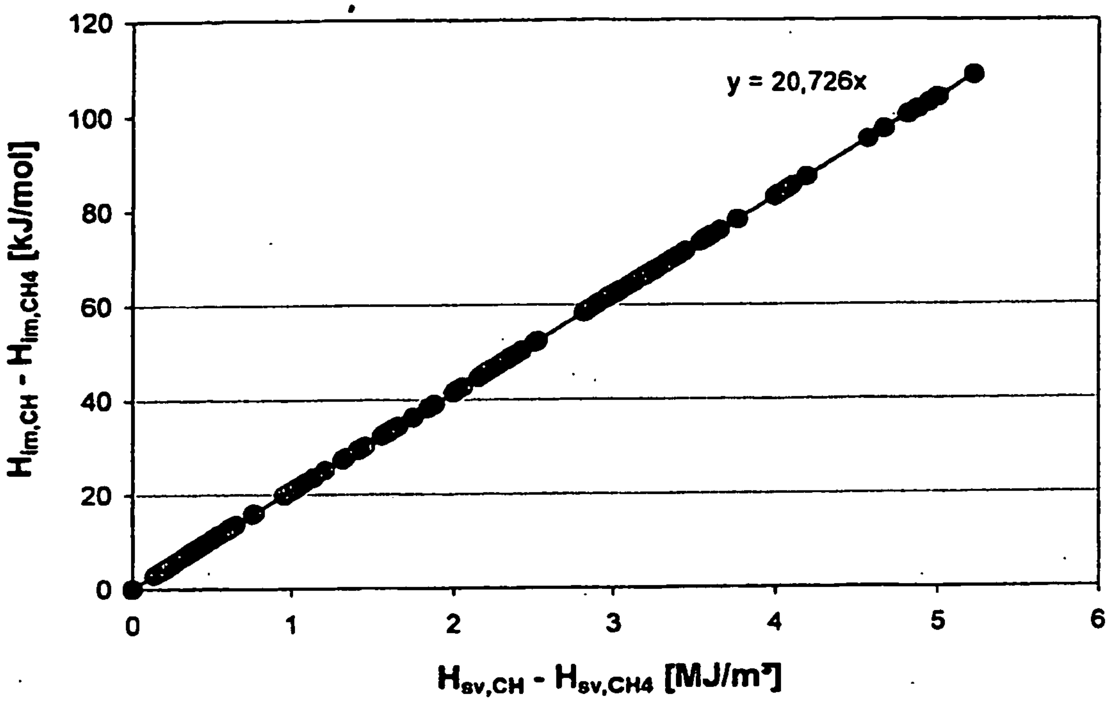
y = 20,726x
120
100
80
60
40
20
0
0
1
2
3
4
5
6
$H_{lm,CH}$ - $H_{lm,CH4}$ [kJ/mol]
$H_{sv,CH}$ - $H_{sv,CH4}$ [MJ/m³]

Fig. 3

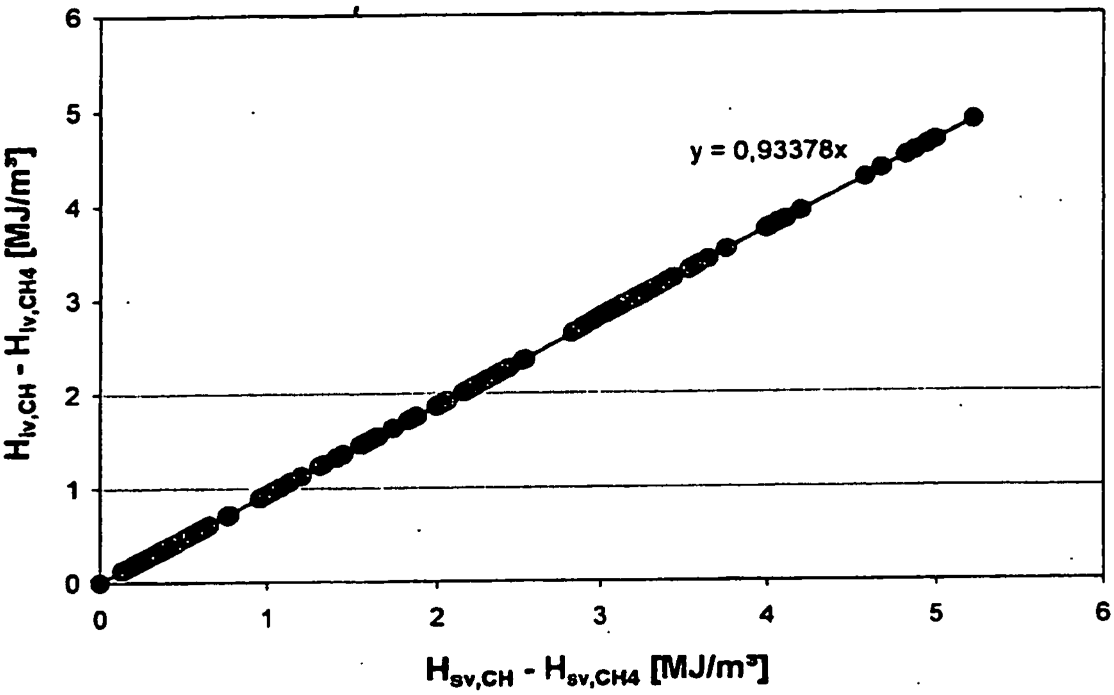

Fig. 4

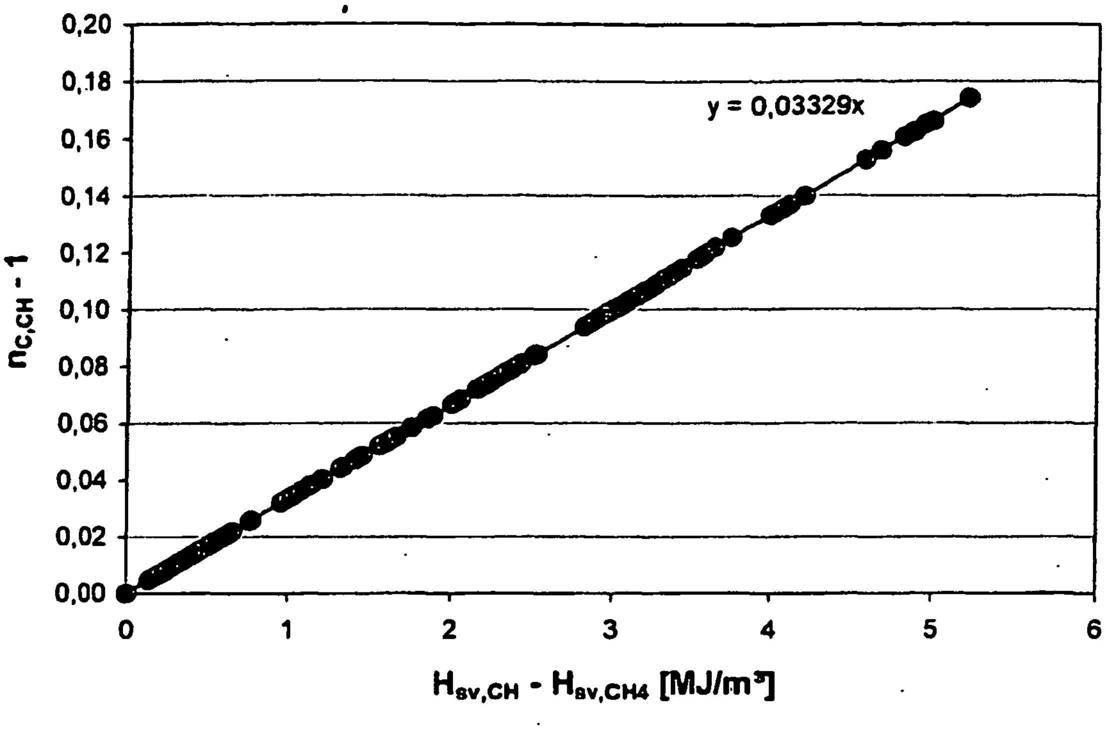
0,20
0,18
0,16
0,14
0,12
0,10
0,08
0,06
0,04
0,02
0,00
y = 0,03329x
$n_{C,CH}$ - 1
0
1
2
3
4
5
6
$H_{sv,CH}$ - $H_{sv,CH4}$ [MJ/m³]

Fig. 5

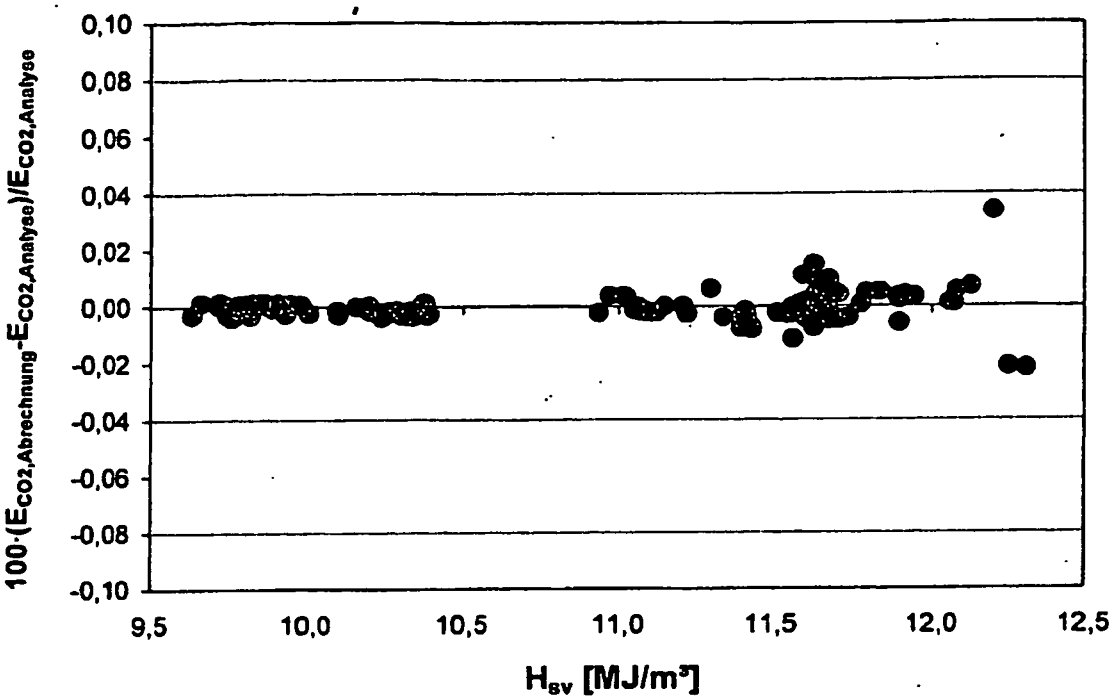
0,10
0,08
0,06
0,04
0,02
0,00
-0,02
-0,04
-0,06
-0,08
-0,10
100·(E_CO2,Abrechnung-E_CO2,Analyse)/E_CO2,Analyse
9,5
10,0
10,5
11,0
11,5
12,0
12,5
H_sv [MJ/m³]

Fig. 6

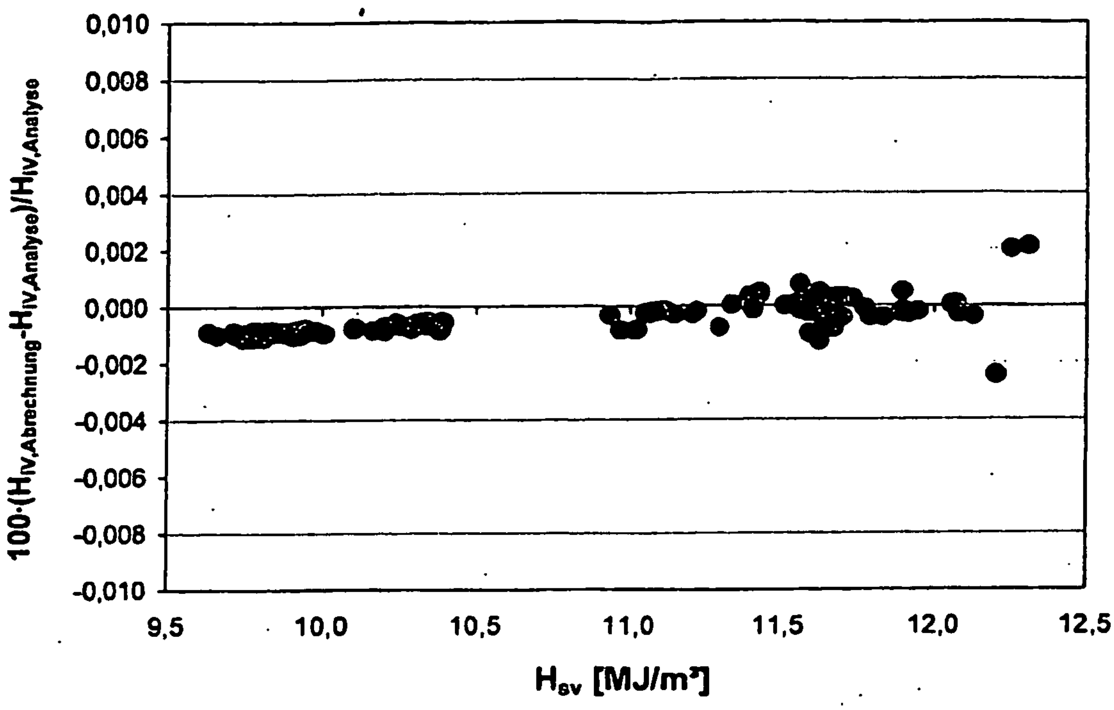
0,010
0,008
0,006
0,004
0,002
0,000
-0,002
-0,004
-0,006
-0,008
-0,010
100·(H_IV,Abrechnung-H_IV,Analyse)/H_IV,Analyse
9,5
10,0
10,5
11,0
11,5
12,0
12,5
H_sv [MJ/m³]

Fig. 7

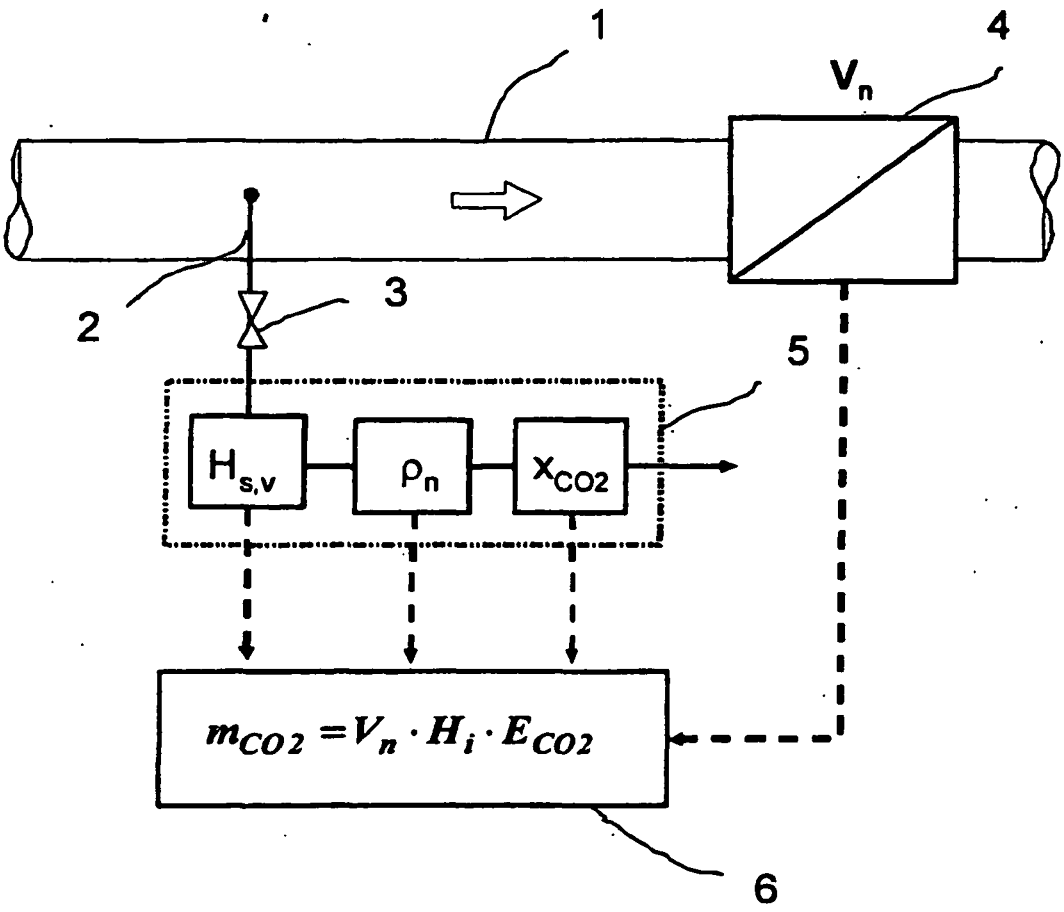

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1715339 A2
- EP 2042850 A2
- EP 0939317 A2


### In der Beschreibung aufgeführte Nicht-Patentliteratur


- *PTB-Anforderung 7.64. Messgeräte für Gas, Brennwertmessgeräte, Ermittlung von Abrechnungsbrennwerten und weiteren Gasbeschaffenheitsdaten mittels Zustandsrekonstruktion,* Dezember 1999